# EUROPÄISCHE PATENTANMELDUNG

(11) **EP 0 590 451 A1**
(43) Veröffentlichungstag der Anmeldung: **06.04.1994**
(21) Anmeldenummer: 93115059.3
(22) Anmeldetag: 18.09.1993
(51) Int. Cl.: C07C 45/46, C07C 49/76, C07C 65/34

(54) **Verfahren zur Acylierung aromatischer Verbindungen**

(30) Priorität: 30.09.1992 DE 4232770
(71) Anmelder: BASF Aktiengesellschaft, D-67063 Ludwigshafen (DE)
(72) Erfinder: Wolf, Hans-Josef, Dr., D-6701 Maxdorf (DE)

(57) **Zusammenfassung**

Verfahren zur Herstellung von Benzoylverbindungen der allgemeinen Formel I 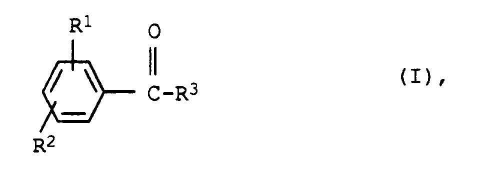
in der
- R¹, R²: Wasserstoff, Hydroxy, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy und
- R³: Wasserstoff, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy
bedeuten, indem man Aromaten der allgemeinen Formel II 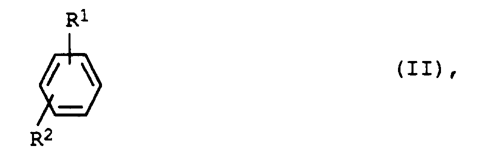
in der R¹ und R² die oben genannten Bedeutungen haben, mit Acylverbindungen der allgemeinen Formel III 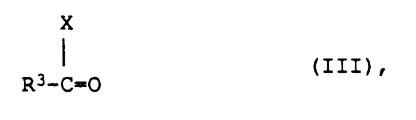
in der R³ die oben genannten Bedeutungen hat und
- X: Halogen oder eine Gruppe R⁴-C=O und
- R⁴: die für R³ angegebenen Bedeutungen hat,
bei Temperaturen von (-60) bis +50°C drucklos in Gegenwart von Fluorwasserstoff, Bortrifluorid und Schwefeldioxid umsetzt.

## Beschreibung

Die vorliegende Erfindung betrifft ein neues Verfahren zur Acylierung aromatischer Verbindungen durch Umsetzung von Aromaten mit Acylverbindungen bei Temperaturen von (-60) bis +50°C und Normaldruck in Gegenwart von Fluorwasserstoff, Bortrifluorid und Schwefeldioxid.

In der EP-A-55 951 wird die Synthese der 2-(4-Ethylbenzoyl)-benzoesäure beschrieben. Katalysator ist ein Gemisch von Fluorwasserstoff und Bortrifluorid. Wegen der geringen Löslichkeit von Bortrifluorid in Fluorwasserstoff entstehen dabei Drücke von z.T. weit über 3 bar.

Deshalb sind Druckapparaturen erforderlich, die wegen der Korrosivität des Katalysators aus teuren Edelstahl-Legierungen gefertigt sein müssen. Ein weiterer Nachteil ist, daß die Reaktion zweiphasig durchgeführt werden muß.

Aus der EP-A-69 598 ist ein Verfahren zur Herstellung von Benzophenonen aus einer aromatischen Verbindung und einer Acylverbindung in Bortrifluorid/Fluorwasserstoff unter Eigendruck oder erhöhtem Druck in Druckapparaturen bekannt.

Der vorliegenden Erfindung lag daher die Aufgabe zugrunde, den zuvor genannten Nachteilen abzuhelfen.

Demgemäß wurde ein neues und verbessertes Verfahren zur Herstellung von Benzoylverbindungen der allgemeinen Formel I 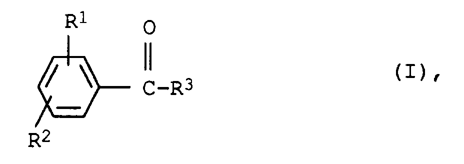
in der
- R¹, R²: Wasserstoff, Hydroxy, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy und
- R³: Wasserstoff, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy
bedeuten, gefunden, welches dadurch gekennzeichnet ist, daß man Aromaten der allgemeinen Formel II 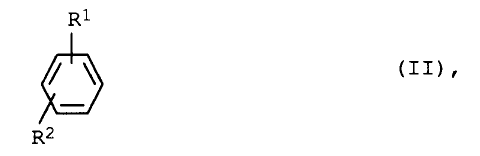
in der R¹ und R² die oben genannten Bedeutungen haben, mit Acylverbindungen der allgemeinen Formel III 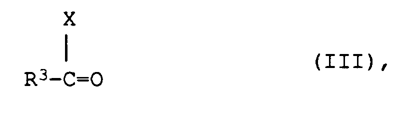
in der R³ die oben genannten Bedeutungen hat und
- X: Halogen oder eine Gruppe R⁴-C=O und
- R⁴: die für R³ angegebenen Bedeutungen hat,
bei Temperaturen von (-60) bis +50°C drucklos in Gegenwart von Fluorwasserstoff, Bortrifluorid und Schwefeldioxid umsetzt.

Das erfindungsgemäße Verfahren läßt sich wie folgt durchführen:
Zu der Acylverbindung III können unter Kühlung Schwefeldioxid, Bortrifluorid und Fluorwasserstoff einkondensiert werden. Zu dieser Mischung kann der Aromat II zugetropft und bei Temperaturen von (-60) bis +50°C, bevorzugt von (-40) bis 30°C und drucklos, also bei Normaldruck (Atmosphärendruck) die Reaktion durchgeführt werden. Danach erwärmt man in der Regel die Reaktionsmischung, bis Schwefeldioxid, Bortrifluorid und Fluorwasserstoff vollständig entfernt sind. Das Rohprodukt kann mit Wasser versetzt, aus dem Reaktionsgefäß gespült und in einem organischen Lösungsmittel aufgenommen werden. Nach Trocknung der organischen Phase wird in der Regel das Lösungsmittel entfernt. Der Rückstand ist in der Regel von hoher Reinheit, kann aber gegebenenfalls durch Umkristallisieren weiter gereinigt werden.

Für die Herstellung der Mischung aus Acylverbindung III, Schwefeldioxid, Bortrifluorid und Fluorwasserstoff kann die Reihenfolge der Zugabe der Einzelstoffe variiert werden. Bei der geringen Löslichkeit von Bortrifluorid in Fluorwasserstoff bei niedrigen Drücken ist es aber vorteilhaft, Acylverbindung III und/oder Schwefeldioxid im Reaktionsgefäß vorzulegen. Bei Temperaturen unterhalb von (-10)°C können dann Fluorwasserstoff und Bortrifluorid zugegeben werden, wobei Bortrifluorid trotz seines Siedepunktes von (-100)°C dann besonders leicht von der Reaktionsmischung aufgenommen wird. Der Aromat kann in einem Lösungsmittel, z.B. einem chlorierten Kohlenwasserstoff wie Dichlormethan, zugesetzt werden. Die Umsetzung kann jedoch auch ohne Lösungsmittel erfolgreich durchgeführt werden. Die Reaktionsdauer liegt zwischen 0,5 und 20 Stunden.

Aromat II und Acylverbindung III können im Molverhältnis von 0,5 : 1 bis 1,5 : 1, bevorzugt 0,9 : 1 bis 1,2 : 1 eingesetzt werden.

Pro Mol der Acylverbindung III werden in der Regel 1 bis 5 Mol Bortrifluorid und pro Mol Bortrifluorid 1 bis 10 Mol Schwefeldioxid verwendet. Schwefeldioxid, im Überschuß angewandt, kann jedoch auch als Reaktionsmedium dienen.

Das Molverhältnis von Fluorwasserstoff zum Bortrifluorid liegt bei 0,1 : 1 bis 2 : 1, bevorzugt 0,2 : 1 bis 1,5 : 1, besonders bevorzugt bei 0,9 : 1 bis 1,1 : 1. Fluorwasserstoff, im Überschuß angewandt, kann jedoch auch als Reaktionsmedium dienen.

Die Substituenten R¹, R², R³, R⁴ und X in den Verbindungen I, II und III haben die folgenden Bedeutungen:
R¹, R², R³ und R⁴
- unabhängig voneinander
- Wasserstoff,
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- C₁- bis C₈-Halogenalkyl, bevorzugt C₁- bis C₄-Halogenalkyl, besonders bevorzugt C₁- bis C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluor- ethyl, 2,2-Dichlor-2-fluorethyl, 2,2,2-Trichlorethyl und Pentafluorethyl,
- Phenyl,
R¹, R² und R³
- zusätzlich
- Phenyl
- Phenoxy,
- ein- bis zweifach durch C₁- bis C₂-Alkyl und/oder Halogen substituiertes Phenyl wie 2-Methylphenyl, 4-Methylphenyl, 2,4-Dimethylphenyl, 2,6-Dimethylphenyl, 2-Ethylphenyl, 4-Ethylphenyl, 2,4-Diethylphenyl und 2,6-Diethylphenyl, 2-Fluor-4-methylphenyl, 2-Chlor-4-methylphenyl, 2-Methyl-4-fluorphenyl und 2-Methyl-4-chlorphenyl, 2-Fluorphenyl, 2-Chlorphenyl, 2-Bromphenyl, 2-Jodphenyl, 4-Fluorphenyl, 4-Chlorphenyl, 4-Bromphenyl, 4-Jodphenyl, 2,4-Difluorphenyl, 2,4-Dichlorphenyl, 2,4-Dibromphenyl, 2,4-Dijodphenyl, 2,6-Difluorphenyl, 2,6-Dichlorphenyl, 2,6-Dibromphenyl und 2,6-Dijodphenyl, bevorzugt 2-Methylphenyl, 4-Methylphenyl, 2,4-Dimethylphenyl, 2,6-Dimethylphenyl, 2-Ethylphenyl, 4-Ethylphenyl, 2,4-Diethylphenyl, 2,6-Diethylphenyl, 2-Chlor-4-methylphenyl, 2-Methyl-4-chlorphenyl, 2-Fluorphenyl, 2-Chlorphenyl, 4-Fluorphenyl, 4-Chlorphenyl, 2,4-Difluorphenyl, 2,4-Dichlorphenyl, 2,6-Difluorphenyl und 2,6-Dichlorphenyl, besonders bevorzugt 2-Methylphenyl, 4-Methylphenyl, 2-Ethylphenyl, 4-Ethylphenyl, 2-Chlor-4-methylphenyl, 2-Methyl-4-chlorphenyl, 2-Fluorphenyl, 2-Chlorphenyl, 4-Fluorphenyl, 4-Chlorphenyl, 2,4-Dichlorphenyl und 2,6-Dichlorphenyl und
- ein- bis zweifach durch C₁- bis C₂-Alkyl und/oder Halogen substituiertes Phenoxy wie 2-Methylphenoxy, 4-Methylphenoxy, 2,4-Dimethylphenoxy, 2,6-Dimethylphenoxy, 2-Ethylphenoxy, 4-Ethylphenoxy, 2,4-Diethylphenoxy und 2,6-Diethylphenoxy, 2-Fluor-4-methylphenoxy, 2-Chlor-4-methylphenoxy, 2-Methyl-4-fluorphenoxy und 2-Methyl-4-chlorphenoxy, 2-Fluorphenoxy, 2-Chlorphenoxy, 2-Bromphenoxy, 2-Jodphenoxy, 4-Fluorphenoxy, 4-Chlorphenoxy, 4-Bromphenoxy, 4-Jodphenoxy, 2,4-Difluorphenoxy, 2,4-Dichlorphenoxy, 2,4-Dibromphenoxy, 2,4-Dijodphenoxy, 2,6-Difluorphenoxy, 2,6-Dichlorphenoxy, 2,6-Dibromphenoxy und 2,6-Dijodphenoxy, bevorzugt 2-Methylphenoxy, 4-Methylphenoxy, 2,4-Dimethylphenoxy, 2,6-Dimethylphenoxy, 2-Ethylphenoxy, 4-Ethylphenoxy, 2,4-Diethylphenoxy, 2,6-Diethylphenoxy, 2-Chlor-4-methylphenoxy, 2-Methyl-4-chlorphenoxy, 2-Fluorphenoxy, 2-Chlorphenoxy, 4-Fluorphenoxy, 4-Chlorphenoxy, 2,4-Difluorphenoxy, 2,4-Dichlorphenoxy, 2,6-Difluorphenoxy und 2,6-Dichlorphenoxy, besonders bevorzugt 2-Methylphenoxy, 4-Methylphenoxy, 2-Ethylphenoxy, 4-Ethylphenoxy, 2-Chlor-4-methylphenoxy, 2-Methyl-4-chlorphenoxy, 2-Fluorphenoxy, 2-Chlorphenoxy, 4-Fluorphenoxy, 4-Chlorphenoxy, 2,4-Dichlorphenoxy und 2,6-Dichlorphenoxy und
R¹ und R²
- zusätzlich
- Phenoxy,
- Hydroxy und
X
- Halogen wie Fluor, Chlor, Brom, Jod, bevorzugt Fluor, Chlor, und Brom, besonders bevorzugt Fluor und Chlor,
- R⁴-C=O.

Die Benzoylverbindungen I eignen sich als Bausteine für einpolymerisierbare Photoinitiatoren (DE-A-4 007 318), als Monomere für Kunststoffe vom Polyetherketontyp, oder als Vorstufen für Anthrachinone (EP-A-55 951).

### Beispiel

### Herstellung von 2-(4-Ethylbenzoyl)-benzoesäure

In ein Teflongefäß mit einem Volumen von 1000 ml, ausgestattet mit einem Teflon-beschichteten Rührer, einem korrosionsbeständigen Innenthermometer und Teflonkühler, wurden 29,6 g (0,2 mol) Phthalsäureanhydrid vorgelegt und unter Rühren bei (-40)°C 410 g (6,4 mol) Schwefeldioxid, 48 g (0,7 mol) Bortrifluorid und 14 g (0,7 mol) Fluorwasserstoff in dieser Reihenfolge einkondensiert. Danach wurden bei (-40)°C 21 g (0,2 mol) Ethylbenzol zugetropft und die Mischung 17 Stunden bei (-40)°C und Normaldruck nachgerührt. Anschließend wurde das Reaktionsgefäß erwärmt, Schwefeldioxid, Fluorwasserstoff und Bortrifluorid in einen Waschturm mit 20-proz. Kalilauge übergetrieben und absorbiert. Der Rückstand wurde mit 150 ml Wasser und 150 ml Dichlormethan aufgenommen, die organische Phase in einem Glasscheidetrichter abgetrennt und über Natriumsulfat getrocknet. Danach wurde das Lösungsmittel entfernt. Man erhielt 2-(4-Ethylbenzoyl)-benzoesäure mit einer Selektivität von 99 % bei einem Umsatz von 87 %.

## Patentansprüche

1. Verfahren zur Herstellung von Benzoylverbindungen der allgemeinen Formel I 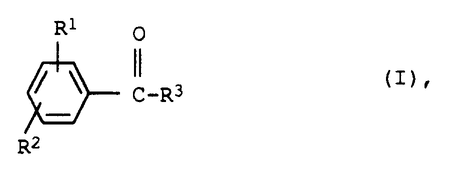 in der
R¹, R² Wasserstoff, Hydroxy, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy und
R³ Wasserstoff, C₁- bis C₈-Alkyl, C₃- bis C₈-Cycloalkyl, C₁- bis C₈-Halogenalkyl, ein gegebenenfalls ein- oder zweifach durch C₁- bis C₂-Alkyl und/oder Halogen substituiertes Phenyl oder Phenoxy
bedeuten, dadurch gekennzeichnet, daß man Aromaten der allgemeinen Formel II 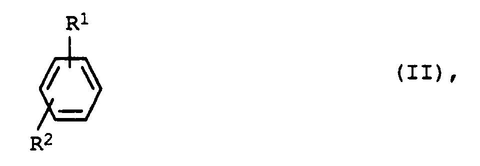 in der R¹ und R² die oben genannten Bedeutungen haben, mit Acylverbindungen der allgemeinen Formel III 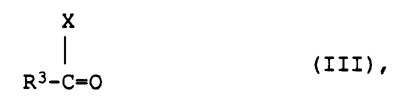 in der R³ die oben genannten Bedeutungen hat und
X Halogen oder eine Gruppe R⁴-C=O und
R⁴ die für R³ angegebenen Bedeutungen hat,
bei Temperaturen von (-60) bis +50°C drucklos in Gegenwart von Fluorwasserstoff, Bortrifluorid und Schwefeldioxid umsetzt.

2. Verfahren zur Herstellung von Benzoylverbindungen I nach Anspruch 1, dadurch gekennzeichnet, daß man die Umsetzung bei Temperaturen von (-40) bis +30°C durchführt.
